(19) Europäisches Patentamt
European Patent Office
Office européen des brevets

(11) EP 0 541 446 B1

(12) FASCICULE DE BREVET EUROPEEN

(45) Date de publication et mention de la délivrance du brevet:
**28.02.1996 Bulletin 1996/09**

(51) Int. Cl.[6]: **C07D 307/93**

(21) Numéro de dépôt: **92402995.2**

(22) Date de dépôt: **05.11.1992**

# (54) Nouveau procédé de préparation de la lactone de l'acide 1R,cis 2,2-diméthyl 3-formyl cyclopropane-1-carboxylique et intermédiaires halogénés

Verfahren zur Herstellung von 1R, cis 2,2,-Dimethyl-3-Formyl-Cyclopropan-1-Carbonsäurelacton und halogenierten Zwischenverbindungen

Procedure for preparing 1R, cis 2,2-dimethyl-3-formyl cyclopropane-1-carboxylic acid lactone, and halogenated intermediates

(84) Etats contractants désignés:
**AT BE CH DE DK ES FR GB GR IE IT LI LU NL PT SE**

(30) Priorité: **08.11.1991 FR 9113776**

(43) Date de publication de la demande:
**12.05.1993 Bulletin 1993/19**

(73) Titulaire: **ROUSSEL UCLAF**
**F-93230 Romainville (FR)**

(72) Inventeurs:
- **Bhatnagar, Neerja**
  **F-91600 Savigny Sur Orge (FR)**
- **Brion, Francis**
  **F-93220 Gagny (FR)**
- **Colladant, Colette**
  **F-93110 Rosny Sous Bois (FR)**

(74) Mandataire: **Vieillefosse, Jean-Claude et al**
**F-93235 Romainville Cédex (FR)**

(56) Documents cités:
**CH-A- 527 152**
**FR-A- 2 396 006**
**GB-A- 2 002 377**

- **HOUBEN-WEYL: 'Methoden der Organischen Chemie, ed. 4, vol. 4/1A (Oxidation, Teil I), "Anorganische Halogen-Verbindungen als Oxidationsmittel", pages 435 - 640' 17 Décembre 1981 , GEORG THIEME VERLAG , STUTTGART, DE**
- **HOUBEN-WEYL: 'Methoden der Organischen Chemie, ed. 4, vol. 4/1B (Oxidation, Teil II), "Wismut- und Vanadin-Verbindungen als Oxidationsmittel", pages 415 - 424' 11 Décembre 1975 , GEORG THIEME VERLAG , STUTTGART, DE**

EP 0 541 446 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention a pour objet un nouveau procédé de préparation de la lactone de l'acide 1R,cis 2,2-diméthyl 3-formyl cyclopropane-1-carboxylique et des intermédiaires halogénés. Un procédé de préparation de composé de formule (I) au départ d'un composé de formule (II) a déjà été décrit dans la demande de brevet anglais 2 002 377. Ce procédé consiste en une cyclodéshydratation conduisant à une énol lactone intermédiaire qui est traitée par l'ozone puis clivée.

L'invention a ainsi pour objet un procédé de préparation du composé de formule (I) :

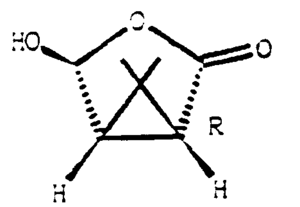

(I)

caractérisé en ce que l'on traite le composé de formule (II) :

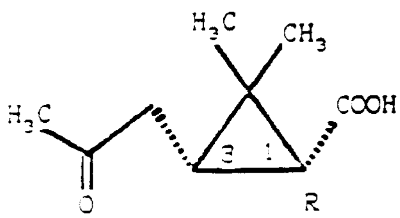

(II)

de configuration 1R,cis, par 2 équivalents au moins d'un halogène choisi dans le groupe constitué par le chlore, le brome et l'iode, pour obtenir :

- soit un composé de formule ($III_1$) :

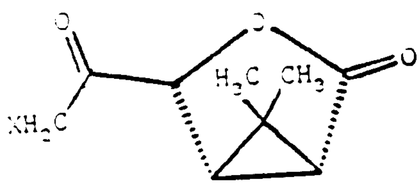

($III_1$)

- soit un composé de formule ($III_2$) :

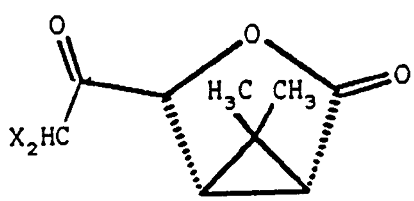

($III_2$)

- soit un composé de formule ($III_3$) :

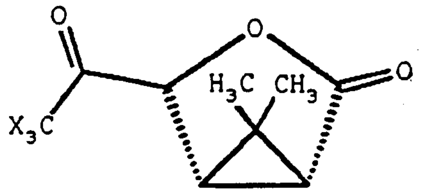

($III_3$)

dans lesquelles X représente un atome d'halogène tel que défini ci-dessus, le cas échéant en mélange avec le composé de formule (IV) :

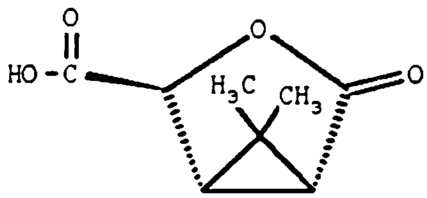

(IV)

poursuit, le cas échéant, l'halogénation du composé de formule ($III_1$) ou ($III_2$) par action d'un excès d'halogène tel que défini ci-dessus, pour obtenir le composé de formule ($III_3$) telle que définie ci-dessus, puis traite le composé de formule ($III_3$), le cas échéant sous la forme du mélange avec le composé de formule (IV), telle que définie ci-dessus, par un agent basique, pour obtenir le composé de formule (IV), existant alors dans le milieu réactionnel sous la forme de son sel correspondant à l'agent basique utilisé, ou de celui de la forme ouverte (IV') :

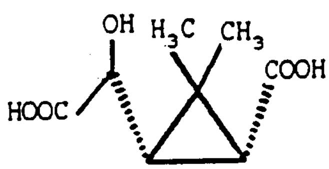

(IV')

acidifie si désiré le milieu réactionnel, pour obtenir l'acide puis soumet ledit acide ou ledit sel à l'action d'un agent oxydant, pour obtenir le composé de formule (I).

L'invention a notamment pour objet un procédé tel que défini précédemment, caractérisé en ce que l'on traite le composé de formule (II) telle que définie précédemment, de configuration 1R,cis, par au moins 4 équivalents d'un halogène tel que défini précédemment, pour obtenir le composé de formule ($III_3$), en mélange avec le composé de formule (IV), puis traite ledit mélange comme indiqué précédemment, pour obtenir le composé de formule (I).

L'invention a plus particulièrement pour objet un procédé tel que défini précédemment, caractérisé en ce que l'halogène utilisé est choisi dans le groupe constitué par le chlore et le brome.

Dans des conditions préférentielles de mise en oeuvre de l'invention,

- on effectue l'halogénation à température ambiante, au sein d'un solvant organique halogéné, lequel peut être par exemple le chlorure de méthylène, le chloroforme, le tétrachlorure de carbone, le dichloréthane, ou un mélange de ces solvants, ou encore un ester organique tel que l'acétate d'éthyle ;
- on traite le brut réactionnel issu de l'halogénation par un agent basique, par exemple un hydroxyde ou un carbonate alcalin ou alcalino-terreux, avant d'isoler le composé halogéné ;

- l'agent basique avec lequel on traite le composé de formule ($III_3$) est choisi dans le groupe constitué par les hydroxydes et les carbonates alcalins, alcalino-terreux et de magnésium et est mis en oeuvre en présence d'eau. Le sel du composé de formule (IV') se forme préférentiellement si l'agent basique est en excès.

L'acide que l'on utilise, le cas échéant, pour libérer l'acide peut être un acide organique ou minéral usuel. On peut citer par exemple l'acide chlorhydrique ou sulfurique.

L'agent oxydant avec lequel l'on traite l'acide ou son sel peut notamment être choisi dans le groupe constitué par les acides hypohalogéneux, les hypohalogénites alcalins, alcalino-terreux et de magnésium, le permanganate de potassium, l'acide chromique, l'acide périodique et les bismuthates alcalins. Il peut encore être, par exemple le dioxyde de manganèse ou un perborate. Un acide hypohalogéneux, notamment l'acide hypochloreux, est plus particulièrement préféré.

Dans des conditions préférées de mise en oeuvre du procédé, l'acide hypohalogéneux utilisé comme oxydant est obtenu in situ à partir d'un hypohalogénite alcalin, alcalinoterreux ou de magnésium placé en milieu acide. L'acide hypochloreux est ainsi obtenu in situ à partir d'hypochlorite de sodium.

L'acide utilisé pour libérer l'acide hypochloreux est de préférence choisi dans le groupe constitué par les acides alcanoïques inférieurs, tels que l'acide acétique ou propionique, ainsi que des solutions de phosphates, borates et acétates de pH suffisamment acide pour libérer l'acide hypochloreux de son sel.

Le composé de formule (II) utilisé au départ du procédé de l'invention est connu par exemple par la publication Agr. Biol. Chem. Vol. 29 N° 8 p. 784 (1965).

A titre purement indicatif, on peut préciser que l'action de l'halogène sur le composé de formule (II) conduit intermédiairement à une forme ouverte de la lactone de formule ($III_1$), ($III_2$) ou ($III_3$) laquelle conduit à ladite lactone par l'action de l'agent basique en milieu aqueux. Il est à noter qu'une certaine quantité de l'acide-lactone de formule (IV) peut également se former en même temps que la lactone et plus particulièrement que la lactone de formule ($III_2$) et ($III_3$).

On peut ainsi schématiser la réaction dans son ensemble comme suit :

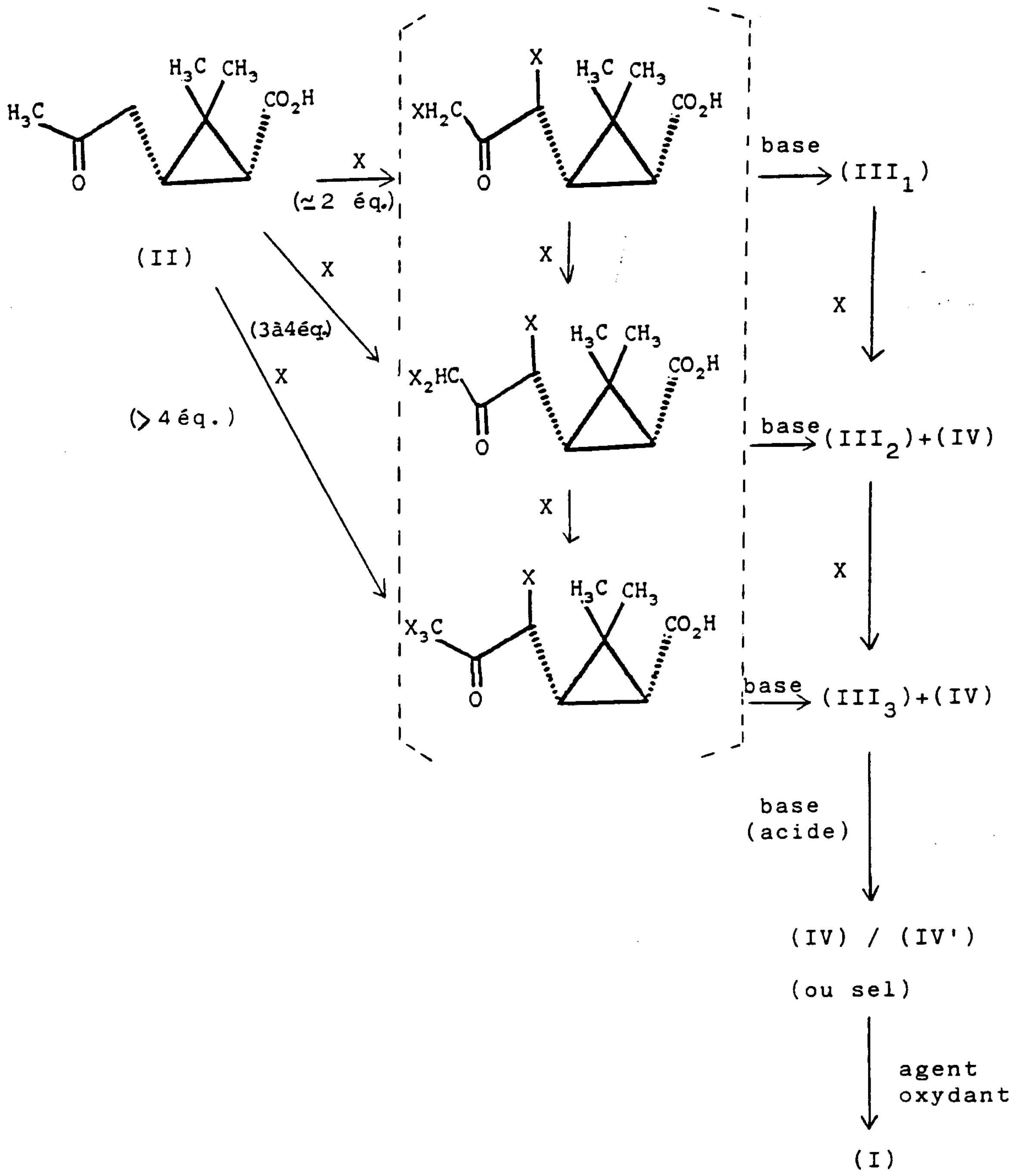


L'invention a enfin pour objet, à titre de produits industriels et notamment à titre d'intermédiaires nécessaires à la mise en oeuvre du procédé de l'invention, les composés de formule ($III_1$), ($III_2$) et ($III_3$) tels que définis précédemment.

Le composé de formule (IV) ainsi que sa forme ouverte (IV') et leurs sels avec les bases sont également des composés nouveaux et constituent l'un des objets de la demande de brevet n° 91 13775 déposée le même jour que la présente demande par la demanderesse et intitulée : "Nouveau procédé de préparation de la lactone de l'acide 1R,cis 2,2-diméthyl 3-formyl cyclopropane-1-carboxylique et intermédiaires".

Le composé de formule (1) est décrit dans le brevet français 1 580 474. Il s'agit d'un important intermédiaire dans la synthèse d'esters bien connus possédant notamment une activité insecticide, ainsi qu'il ressort du brevet français 2 396 006.

Les exemples suivants illustrent l'invention sans toutefois la limiter.

**<u>Exemple 1</u> : (1R-(1alpha,4béta,5alpha))-6,6-diméthyl-4-(tribromo acétyl)-3-oxabicyclo [3.1.0] hexan-2-one et acide (1S-(1alpha,2béta,5alpha)-6,6-diméthyl-4-oxo-3-oxabicyclo [3.1.0] hexane-2-carboxylique.**

On mélange sous gaz inerte 0,51 g d'acide 1R,cis 2,2-diméthyl 3-(2-oxo propyl) cyclopropane-1-carboxylique, 10 $cm^3$ de chloroforme et 10 $cm^3$ de tétrachlorure de carbone, refroidit à +5°C et ajoute lentement 0,7 $cm^3$ (soit 4,5 équivalents) de brome. On agite pendant 18 heures à température ambiante, introduit 15 $cm^3$ d'eau glacée, agite pendant 1 heure puis ajoute 2,12 g de carbonate de potassium et agite à nouveau pendant 1 heure. On verse dans 15 $cm^3$ d'eau, extrait au chlorure de méthylène, sèche la phase organique et évapore à sec. On recristallise le produit brut dans un mélange chlorure de méthylène-éther isopropylique et obtient 0,222 g de dérivé tribromé attendu. F=188-190°C. Une chromatographie sur couche mince du milieu réactionnel a révélé la présence, à côté du dérivé tribromé, du dérivé dibromé correspondant.

On acidifie la phase aqueuse à pH 1 par addition d'acide chlorhydrique 2N et extrait au mélange acétate d'éthyle-méthanol (9-1). On sèche la phase organique et évapore le solvant. On chromatographie le résidu sur silice en éluant au mélange acétate d'éthyle-méthanol (75/25) et obtient 0,2 g de la lactone de l'acide 2,2-diméthyl 3-hydroxy carboxy méthyl cyclopropane-1-carboxylique (ou acide 1S(1alpha,2béta, 5alpha))-6,6-diméthyl-4-oxo-3-oxabicyclo [3.1.0] hexane-2-carboxylique).

<u>Spectre RMN du dérivé tribromé</u> ($CDCl_3$ - 250 MHz)

1,24 et 1,30 : $CH_3$ gem ; 2,17(d,J=6) et 2,31(d,J=6) : $H_1$ et $H_3$ 5,56(s)-O-CH-CO-

**<u>Exemple 2</u>: (1R-(1alpha,4béta,5alpha))-6,6-diméthyl-4-(dibromoacétyl)-3-oxabicyclo [3.1.0] hexan-2-one et acide (1S-(1alpha,2béta,5alpha))-6,6-diméthyl-4-oxo-3-oxabicyclo [3.1.0] hexane-2-carboxylique.**

On mélange sous gaz inerte 0,34 g d'acide 1R,cis 2,2-diméthyl 3-(2-oxo propyl) cyclopropane-1-carboxylique, 6 $cm^3$ de chloroforme et 6 $cm^3$ de tétrachlorure de carbone, refroidit à +5°C et ajoute lentement 0,4 $cm^3$ (soit 4 équivalents) de brome. On agite pendant 6 heures à +5°C puis pendant 16 heures à température ambiante.

On refroidit à +5°C, ajoute 10 $cm^3$ d'eau glacée, agite pendant 1 heure puis ajoute 1,5 g de carbonate de potassium et agite pendant 1 heure. On verse le mélange dans 15 $cm^3$ d'eau et extrait au chlorure de méthylène. On sèche la phase organique et évapore le solvant. On obtient 0,22 g de produit attendu renfermant des traces de dérivé tribromé correspondant.

On acidifie la phase aqueuse à pH 1 par addition d'acide chlorhydrique 2N et extrait à l'acétate d'éthyle. On sèche la phase organique et l'évapore à sec. On chromatographie le résidu sur silice en éluant au mélange chlorure de méthylène-acide acétique (9/1) et obtient 0,1 g de lactone de l'acide 2,2-diméthyl 3-hydroxy carboxy méthyl cyclopropane-1-carboxylique (ou acide (1S-(1alpha,2béta,5alpha))-6,6-diméthyl-4-oxo-3-oxabicyclo [3.1.0] hexane-2-carboxylique.

<u>Spectre RMN du dérivé dibromé</u> ($CDCl_3$ - 250 MHz)

1,24 et 1,25 : $CH_3$ gem ; 2,07(d,J=6) et 2,41(d,J=6) : $H_1$ et $H_3$ ; 4,90(s) : -CO-CH-O- ; 6,38(s) : =C-$CHX_2$

**<u>Exemple 3</u>: (1R-(1alpha,4béta,5alpha))-4-(bromoacétyl)-6,6-diméthyl-3-oxabicyclo [3.1.0] hexan-2-one.**

On mélange sous gaz inerte 0,54 g d'acide 1R,cis 2,2-diméthyl 3-(2-oxo propyl) cyclopropane-1-carboxylique, 5 $cm^3$ de tétrachlorure de carbone et 10 $cm^3$ de chlorure de méthylène. On refroidit à +2, +5°C et ajoute lentement 0,32 $cm^3$ (soit 2 équivalents) de brome.

On agite à température ambiante pendant 1 h 30, puis ajoute 10 $cm^3$ d'eau et poursuit l'agitation pendant 15 minutes. On refroidit à +5°C, ajoute 1,32 g de carbonate de potassium et poursuit l'agitation pendant 1 h à +5°C. On verse le mélange dans 20 $cm^3$ d'eau et extrait au chlorure de méthylène. On sèche la phase organique et évapore le solvant, puis chromatographie le résidu sur silice en éluant au mélange acétate d'éthyle-cyclohexane (7/3). On obtient 0,51 g de produit attendu.

<u>Spectre RMN</u> ($CDCl_3$ - 250 MHz)

1,23(s) : $CH_3$ gem ; 2,05(d,J=6) et 2,34(d,J=6) : $H_1$ et $H_3$ (cyclopropyles cis ; 4,09(d,$J_{AB}$=12,5) et 4,23(d,$J_{AB}$=12,5) : -CO-$CH_2$X ; 4,68(s) -CO-CH-O-CO-

**<u>Exemple 4</u>: (1R-(1alpha,4béta,5alpha))-4-(dibromoacétyl)-6,6-diméthyl-3-oxabicyclo [3.1.0] hexan-2-one.**

On mélange sous gaz inerte 0,125 g de (1R-(1alpha,4béta,5alpha))-4-(bromoacétyl)-6,6-diméthyl-3-oxabicyclo [3.1.0] hexan-2-one obtenu selon le procédé de l'exemple 3 avec 1 $cm^3$ de chlorure de méthylène et 1 $cm^3$ de chloroforme. On ajoute lentement à +25, +30°C, 50 µl de brome puis laisse sous agitation pendant 48 heures à température ambiante. On ajoute de l'eau, décante, extrait au chlorure de méthylène et sèche la phase organique. On évapore le solvant sous pression réduite et obtient 0,199 g de produit attendu brut, sous forme d'une huile.

<u>Spectre RMN</u> ($CDCl_3$ - 250 MHz)

1,24(s) : $CH_3$ gem ; 2,08(d) et 2,41(d) : $H_1$ et $H_3$ ; 4,90(s) : -CO-CH-O- ; 6,38(s) : -CO-$CHX_2$.

**<u>Exemple 5</u>: Lactone de l'acide 1R,cis 2,2-diméthyl 3-hydroxy carboxy méthyl cyclopropane-1-carboxylique ou acide (1S-(1alpha,2béta,5alpha))-6,6-diméthyl-4-oxo-3-oxabicyclo [3.1.0] hexane-2-carboxylique.**

On dissout sous gaz inerte 0,5 g de produit tribromé obtenu comme décrit à l'exemple 1, dans 5 $cm^3$ environ de chlorure de méthylène et ajoute 20 $cm^3$ d'eau, puis 3,4 g de carbonate de potassium. On maintient sous agitation à température ambiante pendant 18 heures puis acidifie à pH 1 par addition d'acide chlorhydrique 2N. On extrait au mélange acétate d'éthyle, méthanol (9/1), sèche la phase organique et évapore le solvant. On obtient 0,2 g de produit attendu brut, que l'on lave par de l'acétate d'éthyle froid. F=177°C. $alpha_D^{20}$= -100° (c=1 % DMF).

<u>Spectre RMN</u> ($CDCl_3$ - 250 MHz)

1,20(s) et 1,26(s) : $CH_3$ gem ; 2,09(d,J=6) et 2,38(m) : $H_1$ et $H_3$/cyclopropyles cis ; 5,12(d,J=6) : -CH-O-

**<u>Exemple 6</u>: Lactone de l'acide 1R,cis 2,2-diméthyl 3-formyl cyclopropane-1-carboxylique ou (1S-(1alpha,2béta,5alpha))-6,6-diméthyl-4-oxo-3-oxabicyclo [3.1.0] hexan-2-ol.**

On mélange 0,8 g d'acide obtenu comme décrit à l'exemple 5 avec 16 $cm^3$ d'eau et 6,3 $cm^3$ de soude 2N. On ajoute à température ambiante 2,4 $cm^3$ d'une solution aqueuse d'hypochlorite de sodium puis quelques gouttes d'acide acétique et maintient sous agitation pendant 1 heure. On ajoute ensuite une solution aqueuse de thiosulfate de sodium jusqu'à disparition du pouvoir oxydant, puis de l'acide chlorhydrique concentré jusqu'à pH 2,5 environ et enfin 10 g de sulfate d'ammonium. On extrait au chlorure de méthylène, sèche la phase organique et évapore à sec. On cristallise le résidu dans du toluène et obtient 0,2 g de produit attendu. F=114,5°C. $alpha_D^{20}$= -101° (c=1 % DMF).

**Revendications**

1. Procédé de préparation du composé de formule (I)

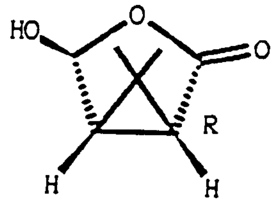

(I)

caractérisé en ce que l'on traite le composé de formule (II) :

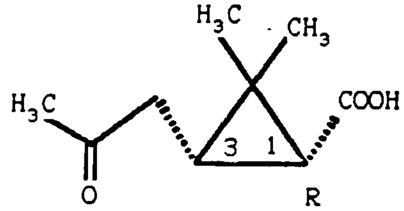

(II)

de configuration 1R,cis, par 2 équivalents au moins d'un halogène choisi dans le groupe constitué par le chlore, le brome et l'iode, pour obtenir :

- soit un composé de formule ($III_1$) :

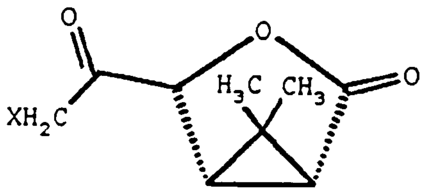

($III_1$)

- soit un composé de formule ($III_2$) :

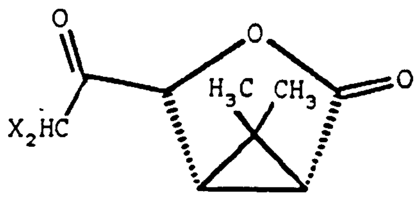

($III_2$)

- soit un composé de formule ($III_3$) :

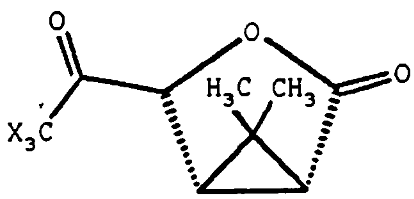

($III_3$)

dans lesquelles X représente un atome d'halogène tel que défini ci-dessus, le cas échéant en mélange avec le composé de formule (IV) :

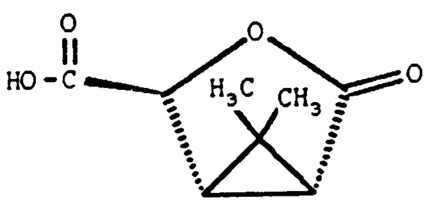

(IV)

poursuit, le cas échéant, l'halogénation du composé de formule ($III_1$) ou ($III_2$) par action d'un excès d'halogène tel que défini ci-dessus, pour obtenir le composé de formule ($III_3$), puis traite le composé de formule ($III_3$), le cas échéant sous la forme du mélange avec le composé de formule (IV), par un agent basique, pour obtenir le composé de formule (IV), existant alors dans le milieu réactionnel sous la forme de son sel correspondant à

l'agent basique utilisé, ou de celui de la forme ouverte (IV') :

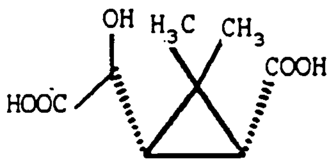

(IV')

acidifie si désiré le milieu réactionnel, pour obtenir l'acide puis soumet ledit acide ou ledit sel à l'action d'un agent oxydant, pour obtenir le composé de formule (I).

2. Procédé selon la revendication 1, caractérisé en ce que l'on traite le composé de formule (II) telle que définie à la revendication 1, de configuration 1R,cis, par au moins 4 équivalents d'un halogène, pour obtenir le composé de formule ($III_3$), en mélange avec le composé de formule (IV), puis traite ledit mélange comme indiqué à la revendication 1, pour obtenir le composé de formule (I).

3. Procédé selon la revendication 1 ou 2, caractérisé en ce que l'halogène utilisé est choisi dans le groupe constitué par le chlore et le brome.

4. Procédé selon l'une quelconque des revendications 1, 2 ou 3, caractérisé en ce que l'on opère au sein d'un solvant organique halogéné ou d'un ester organique.

5. Procédé selon l'une quelconque des revendications 1 à 4, caractérisé en ce que l'on traite le brut réactionnel issu de l'halogénation par un agent basique choisi dans le groupe constitué par les hydroxydes et les carbonates alcalins et alcalino-terreux, avant d'isoler le composé halogéné.

6. Procédé selon la revendication 1, caractérisé en ce que l'agent basique est choisi dans le groupe constitué par les hydroxydes et les carbonates alcalins, alcalino-terreux et de magnésium et est mis en oeuvre en présence d'eau.

7. Procédé selon la revendication 1, caractérisé en ce que l'agent oxydant est choisi dans le groupe constitué par les acides hypohalogéneux, les hypohalogénites alcalins, alcalino-terreux et de magnésium, le permanganate de potassium, l'acide chromique, l'acide périodique et les bismuthates alcalins.

8. Procédé selon la revendication 1 ou 7, caractérisé en ce que l'agent oxydant est un acide hypohalogéneux.

9. Procédé selon la revendication 8, caractérisé en ce que l'acide hypohalogéneux est obtenu in situ à partir d'un hypohalogénite alcalin, alcalino-terreux ou de magnésium placé en milieu acide.

10. Procédé selon la revendication 9, caractérisé en ce que l'agent oxydant est l'acide hypochloreux.

11. Procédé selon la revendication 9 ou 10, caractérisé en ce que l'acide hypochloreux est obtenu in situ à partir d'hypochlorite de sodium placé en milieu acide.

12. Procédé selon la revendication 9 ou 11, caractérisé en ce que l'acide est choisi dans le groupe constitué par les acides alcanoïques inférieurs et les solutions de phosphates, borates et acétates de pH suffisamment acide pour libérer l'acide hypochloreux de son sel.

13. Procédé selon la revendication 12, caractérisé en ce que l'acide est l'acide acétique ou propionique.

14. A titre de composés industriels, les composés de formule ($III_1$), ($III_2$) et ($III_3$) tels que définis à la revendication 1.

## Claims

1. Preparation process for the compound of formula (I)

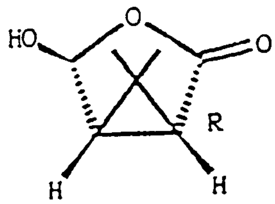

(I)

characterized in that the compound of formula (II):

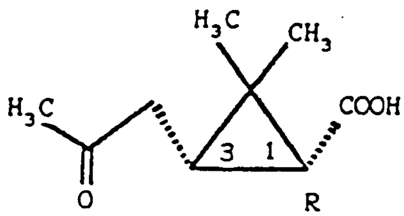

(II)

of 1R,cis configuration, is treated with at least 2 equivalents of a halogen chosen from the group constituted by chlorine, bromine and iodine, in order to obtain:

- either a compound of formula ($III_1$):

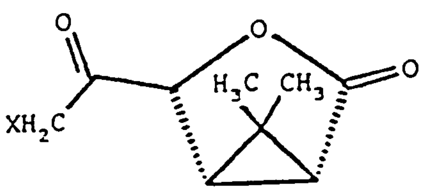

($III_1$)

- or a compound of formula ($III_2$):

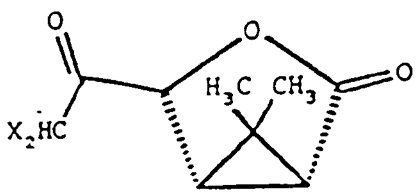

($III_2$)

- or a compound of formula ($III_3$):

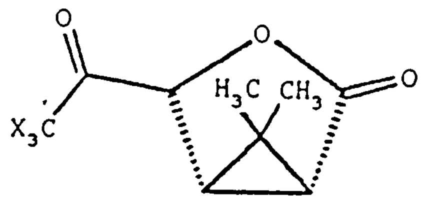

($III_3$)

in which X represents a halogen atom as defined above, if appropriate in a mixture with the compound of formula (IV):

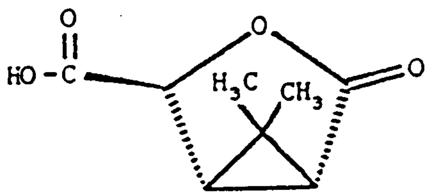

(IV)

if appropriate, the halogenation of the compound of formula ($III_1$) or ($III_2$) is continued by the action of an excess of halogen as defined above, in order to obtain the compound of formula ($III_3$), then the compound of formula ($III_3$) is treated, if appropriate in the form of a mixture with the compound of formula (IV), by a basic agent, in order to obtain the compound of formula (IV), existing in that case in the reaction medium in the form of its salt corresponding to the basic agent used, or of that of the open form (IV'):

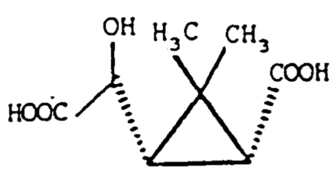

(IV')

if desired the reaction medium is acidified, in order to obtain the acid, the said acid or the said salt is then subjected to the action of an oxidizing agent, in order to obtain the compound of formula (I).

2. Process according to claim 1, characterized in that the compound of formula (II) as defined in claim 1, of 1R,cis configuration, is treated with at least 4 equivalents of a halogen, in order to obtain the compound of formula ($III_3$), in a mixture with the compound of formula (IV), then said mixture is treated as indicated in claim 1, in order to obtain the compound of formula (I).

3. Process according to claim 1 or 2, characterized in that the halogen used is chosen from the group constituted by chlorine and bromine.

4. Process according to any one of claims 1, 2 or 3, characterized in that the operation takes place in a halogenated organic solvent or an organic ester.

5. Process according to any one of claims 1 to 4, characterized in that the crude reaction medium deriving from the halogenation is treated with a basic agent chosen from the group constituted by alkaline and alkaline-earth hydroxides and carbonates, before isolating the halogenated compound.

6. Process according to claim 1, characterized in that the basic agent is chosen from the group constituted by alkaline, alkaline-earth and magnesium hydroxides and carbonates and is used in the presence of water.

7. Process according to claim 1, characterized in that the oxidizing agent is chosen from the group constituted by hypohalogenous acids, alkaline, alkaline-earth and magnesium hypohalogenites, potassium permanganate, chromic acid, periodic acid and the alkaline bismuthates.

8. Process according to claim 1 or 7, characterized in that the oxidizing agent is a hypohalogenous acid.

9. Process according to claim 8, characterized in that the hypohalogenous acid is obtained in situ from an alkaline, alkaline-earth or magnesium hypohalogenite placed in acid medium.

10. Process according to claim 9, characterized in that the oxidizing agent is hypochlorous acid.

11. Process according to claim 9 or 10, characterized in that the hypochlorous acid is obtained in situ from sodium hypochlorite placed in acid medium.

12. Process according to claim 9 or 11, characterized in that the acid is chosen from the group constituted by lower alkanoic acids and solutions of phosphates, borates and acetates of sufficiently acid pH to release the hypochlorous acid from its salt.

13. Process according to claim 12, characterized in that the acid is acetic or propionic acid.

14. As industrial compounds, the compounds of formula ($III_1$), ($III_2$) and ($III_3$ as defined in claim 1.

**Patentansprüche**

1. Verfahren zur Herstellung einer Verbindung der Formel (I)

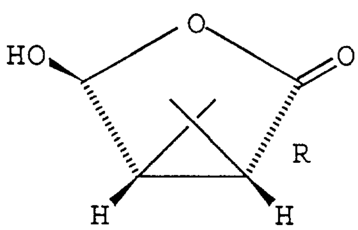

(I)

dadurch gekennzeichnet, daß man die Verbindung der Formel (II)

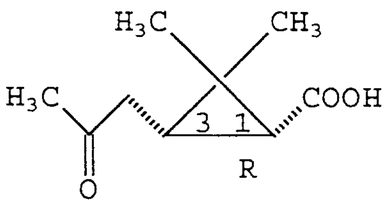

(II)

der Konfiguration 1R,cis mit 2 Äquivalenten von mindestens einem Halogen, gewählt aus der von Chlor, Brom und Iod gebildeten Gruppe, behandelt, um zu erhalten:

- entweder eine Verbindung der Formel ($III_1$)

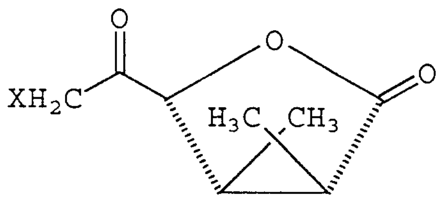

($III_1$)

- oder eine Verbindung der Formel ($III_2$)

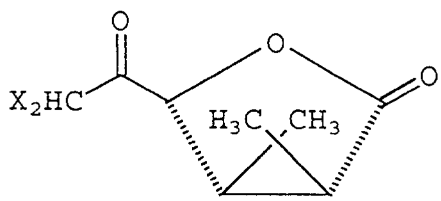

($III_2$)

- oder eine Verbindung der Formel ($III_3$)

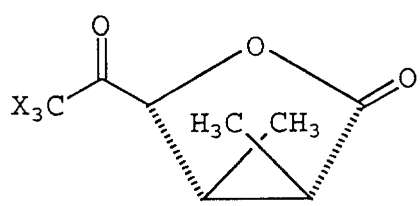

($III_3$)

in denen X ein wie vorstehend definiertes Halogenatom darstellt, gegebenenfalls in Mischung mit der Verbindung der Formel (IV)

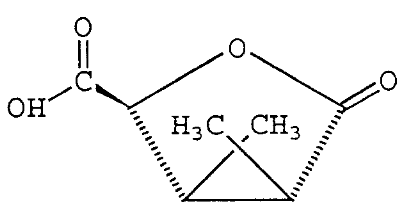

(IV)

und man anschließend gegebenenfalls die Halogenierung der Verbindung der Formeln ($III_1$) oder ($III_2$) durch Einwirkung eines Überschusses von wie vorstehend definiertem Halogen vornimmt, um die Verbindung der Formel ($III_3$) zu erhalten, und man danach die Verbindung der Formel ($III_3$), gegebenenfalls in Form der Mischung mit der Verbindung der Formel (IV), mit einem basischen Mittel behandelt, um die Verbindung der Formel (IV) zu erhalten, die demnach in dem Reaktionsmedium in Form ihres dem verwendeten basischen Mittel entsprechenden Salzes oder in der offenen Form (IV')

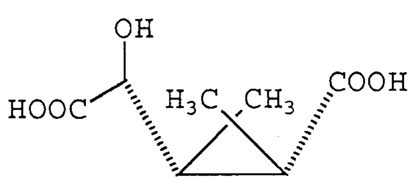

(IV')

existiert, und man gewünschtenfalls das Reaktionsmedium ansäuert, um die Säure zu erhalten, und man

schließlich die genannte Säure oder das Salz der Einwirkung eines Oxidationsmittels unterzieht, um die Verbindung der Formel (I) zu erhalten.

2. Verfahren nach Anspruch 1, dadurch gekennzeichnet, daß man die wie in Anspruch 1 definierte Verbindung der Formel (II) mit der Konfiguration 1R,cis mit mindestens 4 Äquivalenten eines Halogens behandelt, um die Verbindung der Formel ($III_3$) in Mischung mit der Verbindung der Formel (IV) zu erhalten, und man anschließend die genannte Mischung wie in Anspruch 1 angegeben behandelt, um die Verbindung der Formel (I) zu erhalten.

3. Verfahren nach Anspruch 1 oder 2, dadurch gekennzeichnet, daß das verwendete Halogen aus der durch Chlor und Brom gebildeten Gruppe gewählt wird.

4. Verfahren nach irgendeinem der Ansprüche 1, 2 oder 3, dadurch gekennzeichnet, daß man in einem halogenierten organischen Lösungsmittel oder in einem organischen Ester arbeitet.

5. Verfahren nach irgendeinem der Ansprüche 1 bis 4, dadurch gekennzeichnet, daß man das aus der Halogenierung stammende rohe Reaktionsprodukt vor der Isolierung der halogenierten Verbindung mit einem basischen Mittel behandelt, gewählt aus der durch die Hydroxide und Carbonate von Alkali- und Erdalkalimetallen gebildeten Gruppe.

6. Verfahren nach Anspruch 1, dadurch gekennzeichnet, daß das basische Mittel aus der durch die Hydroxide und Carbonate von Alkali-, Erdalkalimetallen und Magnesium gebildeten Gruppe gewählt und in Anwesenheit von Wasser gearbeitet wird.

7. Verfahren nach Anspruch 1, dadurch gekennzeichnet, daß das Oxidationsmittel aus der durch die Hypohalogensäuren, die Hypohalogenite von Alkali-, Erdalkalimetallen und Magnesium, Kaliumpermanganat, Chromsäure, Periodsäure und die Alkalibismutate gebildeten Gruppe gewählt wird.

8. Verfahren nach Anspruch 1 oder 7, dadurch gekennzeichnet, daß das Oxidationsmittel eine Hypohalogensäure ist.

9. Verfahren nach Anspruch 8, dadurch gekennzeichnet, daß die Hypohalogensäure ausgehend von einem Hypohalogenit eines Alkali-, Erdalkalimetalles oder von Magnesium in situ im sauren Medium erhalten wird.

10. Verfahren nach Anspruch 9, dadurch gekennzeichnet, daß das Oxidationsmittel Hypochlorsäure ist.

11. Verfahren nach Anspruch 9 oder 10, dadurch gekennzeichnet, daß die Hypochlorsäure ausgehend von Natriumhypochlorit erhalten wird, das man in ein saures Medium bringt.

12. Verfahren nach Anspruch 9 oder 11, dadurch gekennzeichnet, daß die Säure aus der durch die niederen Alkansäuren und die Lösungen von Phosphaten, Boraten und Acetaten gebildeten Gruppe gewählt wird, die einen ausreichend sauren pH-Wert aufweisen, um die Hypochlorsäure aus ihrem Salz freizusetzen.

13. Verfahren nach Anspruch 12, dadurch gekennzeichnet, daß die Säure Essigsäure oder Propionsäure ist.

14. Als industrielle Verbindungen die wie in Anspruch 1 definierten Verbindungen der Formeln ($III_1$), ($III_2$) und ($III_3$).